# EUROPEAN PATENT SPECIFICATION

(11) **EP 0 416 730 B1**
(45) Date of publication and mention of the grant of the patent: **22.05.1996**
(21) Application number: 90307981.2
(22) Date of filing: 20.07.1990
(51) Int. Cl.: G01N 33/547, G01N 33/552, G01N 29/18, C12Q 1/68, G01N 33/68

(54) **Substrate preparation for chemical-species-specific binding**
Herstellung einer Festphase, geeignet für spezifische Bindung chemischer Substanzen
Préparation d'une phase solide liant spécifiquement des substances chimiques

(30) Priority: 08.09.1989 US 404721
(43) Date of publication of application: 13.03.1991
(73) Proprietor: Hewlett-Packard Company, Palo Alto, California 94304 (US)
(72) Inventor: Tom-Moy, May, San Carlos, California 94070 (US); Meyerholtz, Carl Alan, Cupertino, California 94070 (US)
(74) Representative: Colgan, Stephen James

(56) References cited:
- EP-A- 0 138 297
- EP-A- 0 139 489
- EP-A- 0 215 669
- EP-A- 0 246 846
- WO-A-87/04794
- ANALYTICAL CHEMISTRY vol. 59, no. 23, 1 December 1987, pages 2760 - 2763; MURAMATSU ET AL.: 'Piezoelectric crystal biosensor modified with protein A for determination of immunoglobulins'
- ANALYTICAL CHEMISTRY vol. 61, no. 11, 1 June 1989, pages 1227 - 1230; DAVIS ET AL.: 'Continuous liqiud-phase piezoelectric biosensor for kinetic immunoassays'

## Description

### BACKGROUND OF THE INVENTION

The present invention relates to analytical chemistry and, more particularly, to devices and methods which provide for selectively binding chemical species to a substrate. A major objective of the present invention is to provide for more convenient and effective chemical binding to a substrate used in the context of a mass biosensor.

Preservation of the environment requires that the amounts of various pollutants on land and in water be monitored. Laboratories monitoring these pollutants are charged with measuring microquantities of many different chemicals. Mass biosensors provided a valuable tool in this application, as well as in medical and other applications.

Mass biosensors are used to measure microquantities of biological components and have the potential for detecting trace amounts of biological and chemical components. One type of mass biosensor uses a piezoelectric crystal as an acoustic waveguide. An input transducer generates a periodic acoustic wave from a periodic electrical input signal. The acoustic wave propagates through the crystal to an output transducer which converts the received acoustic wave to an electrical output signal. The acoustic wave undergoes a change in propagation velocity which corresponds to the mass bound of the surface of the crystal. By monitoring the frequency or relative phases of the input and output electrical signals, the mass changes at the surface of the crystal can be measured.

To measure the amount of a specific chemical component in a sample solution, the surface of the crystal must be prepared to bind that component selectively. In one approach, a scientist obtains an unmodified crystal and prepares it shortly before component measurement so that it acquires an affinity for the component of interest. For example, an antibody can be bound to a crystal surface to prepare the mass biosensor to measure the amount of the corresponding antigen.

Heretofore, piezoelectric crystal biosensors were constructed so that antibody proteins which bind antigens or antibody-binding proteins which bind antibodies were bound directly to the surface. A major drawback of this method is the extensive amount of time necessary to bind these proteins to the surface, a procedure taking many hours. In some cases, the preparation procedures take 24 hours or more.

Another drawback is that the shelf life of the sensors is limited by the stability of the proteins bound on its crystal surface. Antibodies and antibody-binding proteins require cold storage and lose binding activity with time. Still another drawback is that some of the proteins can be attached to the surface in an orientation that obscures binding sites for the compound of interest. Additionally, the procedures for immobilizing the proteins on the surface exposes them to chemicals which can lower binding activity by affecting functional groups at the binding sites. Furthermore, the procedure can require additional modifications for each specific protein system. Yet another problem is the high degree of nonspecific adsorption on the surface: many molecules in solution will bind to the surface by means of weak electrostatic and hydrogen bonds. In a mass biosensor, this nonspecific binding effects the measurement, limiting the sensitivity of the instrument and its analytical and clinical usefulness.

What is needed is a quick and convenient procedure for customizing a sensor to bind chemicals of interest for diverse applications. Additionally needed is a sensor with minimal nonspecific binding so that it can be used in detecting trace quantities of chemicals of interest.

EP-A-0138297 describes immuno-agglutination particle suspensions comprising antibody molecules attached to a carboxylate derivatized polymer core. The antibody is linked to the core through an avidin-biotin bridge. Avidin is joined by an amide bond to carboxyl groups on the core, and biotin is linked by an amide bond to amino groups on the antibody molecule. The use of such a bridge system allows controlled amounts of antibody to be attached to the particles, thereby improving reproducibility and reducing false positive results due to excess antibody on the particles causing agglutination and precipitation.

EP-A-0139489 describes a sandwich hybridisation method for nucleic acid detection. The method uses a biotinylated nucleic acid probe for detecting base sequences of interest in nucleic acids. The biotinylated probe hybridises to an immobilised portion of a DNA strand. An avidin-conjugated micro-particle attaches to the biotin on the hybridized probe. The nuclei acid hybrid pair can then be isolated by techniques such as centrifugation.

W087/04794 describes immuno-agglutination latex particle suspensions similar to those of EP-A-0138297, but with noncovalent bonding of the avidin to the surface of the particle and with provision of a linker group R between the biotin and the antibody.

EP-A-0215669 describes a piezoelectric acoustic mass sensor device. The electrode surface of the piezoelectric crystals are treated with an organosilane coupling agent, followed by glutaraldehyde and an antibody to the selected analyte.

H. Muramatsu *et al,* in Analytical Chemistry vol. 59, pp 2760-2763 (1987) describe piezoelectric crystal mass sensors modified with protein A for the determination of immunoglobulins. The crystal substrate surface is first modified with (γ-aminopropyl) triethoxysilane. The substrate is then air-dried and treated with a glutaraldehyde solution. The Protein A is then immobilised onto the electrode by means of the surface aldehyde and, after immobilisation, the remaining unreacted aldehyde is blocked with glycine.

The present invention provides a method of rendering a substrate surface of a mass sensor specific for the binding of a selected substance, said method comprising the steps of:
attaching an organosilane coupling agent to said substrate surface;
attaching a first binding layer to said organosilane coupling agent, said first binding layer having first layer high affinity binding sites available; and subsequently
attaching a second binding layer to said first layer high affinity binding sites through reciprocal high affinity binding sites on said second binding layer, said second binding layer also having second layer binding sites available for the binding of said selected substance.

The present invention also provides a mass sensor for measuring the mass of an analyte in solution, said sensor comprising:
a substrate having a surface to which said analyte is to be attached;
an organosilane coupling layer bound to said surface, said organosilane coupling layer having a functional group;
a ligand-binding layer attached to said surface by means of said functional group said ligand-binding layer having high affinity ligand-binding sites for binding a ligand; and
a ligand-bearing layer strongly adhering through said ligand to said ligand-binding sites of said ligand-binding layer, said ligand-bearing layer having second binding sites that are chemically selective for said analyte, whereby said analyte is attached to said surface by means of said ligand-bearing layer, said ligand-binding layer and said organosilane coupling layer so that said mass can be determined by said sensor.

Preferably, the mass sensor is a surface transverse wave (STW) mass sensor.

As an example, avidin can be coupled to a silica substrate, a biotinylated antibody can be attached to the avidin and biotin can be added to block unoccupied active sites. This composite surface will bind tightly to antigen with minimal nonspecific adsorption.

As indicated, avidin is favoured material for the ligand-binding layer and biotinylated antibodies are appropriate for the ligand-bearing layer. A biotinylated antibody is composed of an antibody bound to biotin directly or via a spacer molecule. Biotin has one avidin-binding site per molecule and avidin has four biotin-binding sites per molecule. Avidin and biotin bind tightly to one another through their reciprocal binding sites, even when each is in turn conjugated to other molecules, as long as the high affinity binding sites are not blocked.

The present invention provides a device which, when immersed in a liquid, binds selected chemicals. The surface of the device has overlaying layers composed of ligand-binding substance and ligand-conjugated compound, the latter having strong binding affinity toward the chemicals of interest. The ligand-binding substance can be bound to the substrate using a coupling agent. Nonspecific binding can be controlled by pretreatment with blocking agents, buffers, or other regimen. Blocking agents are chemicals that bind to the device and sterically inhibit the nonspecific binding from weak bond formation. Buffers of appropriate pH can inhibit nonspecific binding by neutralizing charged sites on the surface.

A preferred mass biosensor provided by the present invention includes an electric signal generator, an electro-acoustic input transducer, a piezoelectric crystal waveguide, an electro-acoustic output transducer, and means for measuring phase changes between the electric signals from the generator and output transducer. In this case, the waveguide is immersed in a liquid, a signal is applied, and the phase or frequency of the output signal from the waveguide is monitored. Mass changes on the surface of the waveguide affect the output signal and the mass or concentration of the analyte can be calculated from these data.

The present invention provides for preparation of the measurement surface in two phases to obviate problems of excessive preparation time and product instability. In accordance with the preferred embodiment, the first phase of preparation involves attaching avidin to the sensor surface. This phase, which is time consuming, can be accomplished efficiently by a manufacturer of the device. Avidin retains its binding activity at room temperature so that the avidin-coated device has a relatively long shelf life and, for manufacturing purposes, can be packaged and distributed without cold storage at considerable cost saving. A further advantage to the manufacturer is that avidin, which is a constituent of egg whites, can be abundantly supplied at low cost. Still another advantage for applications requiring sterile conditions is that the avidin-modified sensor can be sterilized since avidin withstands temperatures up to 120°C.

The second phase involves attaching a biotinylated compound to the avidin and blocking weak binding sites on the surface. This phase can be accomplished with ease by the user in about 30 minutes. Although biotinylated antibody is less stable than avidin, this component need not be added to the surface until just prior to use. Another advantage to the user is that the second phase of preparation requires no expensive apparatus or chemicals. Further, this phase of the procedure is accomplished at room temperature under physiological conditions so that the binding activity of the biotinylated compounds is not compromised by harsh chemicals or high temperatures. Of great convenience to the user is the fact that biotinylated compounds are available commercially or can be prepared following published procedures. Thus, the present invention significantly enhances the convenience with which mass biosensors can be customized to assess the amounts of selected chemicals.

An important feature of the present invention is that it can reduce nonspecific binding. Free, active binding regions on the silica surface are sterically blocked by bound avidin. Free, unbound sites on avidin are blocked in the final wash step with biotin. Since biotin is small and of low molecular weight, it has access to unbound binding sites that may otherwise be inaccessible to larger blocking agents because of steric hindrance.

Yet another feature of the invention is the multiplication of binding sites. Since a single molecule of avidin has four biotin binding sites, when a silica surface is overlaid with avidin, the number of potential binding sites for biotinylated derivatives is fourfold the number of binding sites for avidin. When using biotinylated antibodies for the next layer, each of which contains two antigen binding sites, potential binding sites for the antigenic species are amplified again. These and other features and advantages of the present invention will be apparent from the description below with reference to the following drawings.

### BRIEF DESCRIPTION OF THE DRAWINGS

FIGURE 1 is a schematic view of a sensor utilizing a surface transverse wave device with a chemically modified top surface.

FIGURE 2 is a schematic illustration of the process for immobilizing avidin to a substrate.

FIGURE 3 is a schematic illustration of the process for attaching a biotinylated antibody to an avidin-coated substrate.

### DESCRIPTION OF THE PREFERRED EMBODIMENTS

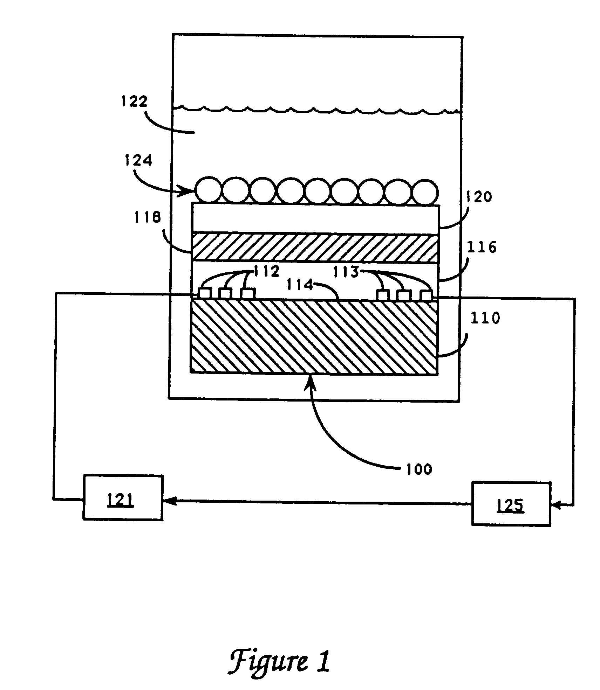

In accordance with the present invention, a mass biosensor 100 comprises a surface transverse wave (STW) device 110 with input transducers 112 and output transducers 113 on its top surface 114, as shown in FIG. 1. A coupling layer 116, formed from an organosilane chemical, covers top surface 114. Coupling layer 116 serves as a site of attachment for an avidin layer 118, which binds tightly to a biotinylated antibody layer 120. Biotinylated antibody layer 120 can be replaced by other biotinylated compounds as appropriate for the particular application of the biosensor. A periodic signal produced by a signal generator 121 passes through the input transducers 112, where it is converted to a periodic acoustic wave and propagated along the surface transverse wave device 110, to the output transducers 113.

When the sensor is immersed in sample 122, antigen 124 binds to biotinylated antibody layer 120. The binding of antigen 124 causes the mass bound to surface 114 to change. This change is reflected in a change in propagation velocity of the acoustic wave between transducers 112 and 113. At the output transducers 113, the periodic acoustic wave is converted into a periodic electrical signal which passes to the signal processor 125. The signal indicating the frequency or relative phases is then used to calculate the amount of antigen 123 in sample 122.

After taking the measurement, antigen 124 can be stripped from biotinylated antibody layer 120 by washing with a high ionic strength salt solution such as 1.5 M NaCl. Different stripping methods are appropriate for use with other biotinylated conjugates. After the antigen is stripped from the antibody layer the biosensor can be reused.

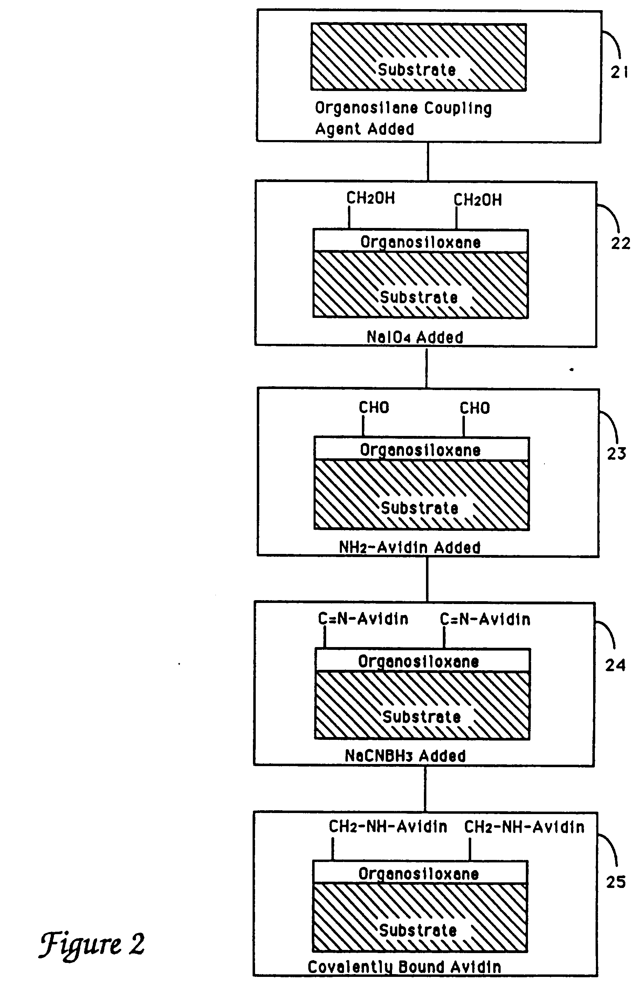

A schematic illustration of the process for immobilizing avidin to a quartz substrate is presented in Figure 2. The substrate is pretreated by sputter deposition of SiO₂ to a thickness of 50nm (500 Angstroms) to provide a number of active hydroxyl (-OH) groups. In the first step 21, an organosilane coupling agent is added to the substrate although other appropriate coupling agents could be used. The organosilane coupling agent has a general formula of RₙSiX₍₄₋ₙ₎. X represents a hydrolyzable group, for example, alkoxy, acyloxy, amine, or chlorine. R represents a nonhydrolyzable organic radical containing a primary alcohol group. The integer n can be 1, 2, or 3 but is most typically 1. The R and X groups are bound to the central silicon atom (Si). The organosilane coupling agent is 3-glycidoxypropyltrimethoxysilane (GOPS). The chemistry for this step is as described in "Silane Coupling Agent Chemistry", Barry Arkles, *Petrarch Systems Register and Review,* R. Anderson, B. Arkles, and G.L. Larson, Eds., 1987. As a result, an organodihydroxysiloxane coats the substrate. In the second step 22, addition of a suitable oxidizing agent, such as sodium periodate, converts the primary alcohol groups on the organic radicals to reactive aldehydes. In the third step 23, avidin is added and the aldehydes react with a primary amine on avidin to form a labile Schiff's base. In the fourth step 24, a suitable reducing agent is added, such as sodium cyanoborohydride, which converts the labile complex to a stable reduction product represented in 25.

Streptavidin, a bacterial protein, can substitute for avidin since it has similar biotin binding properties. Avidin is a glycosylated protein, which means it has an abundance of sugar residues attached, providing potential sites for nonspecific binding. Streptavidin is a nonglycosylated neutral protein which may be useful in certain applications for alleviating nonspecific binding. Modified forms of avidin or streptavidin, as, for example, by acetylation of succinylation, or genetically engineered avidin or streptavidin can also be used so long as the biotin binding sites remain intact.

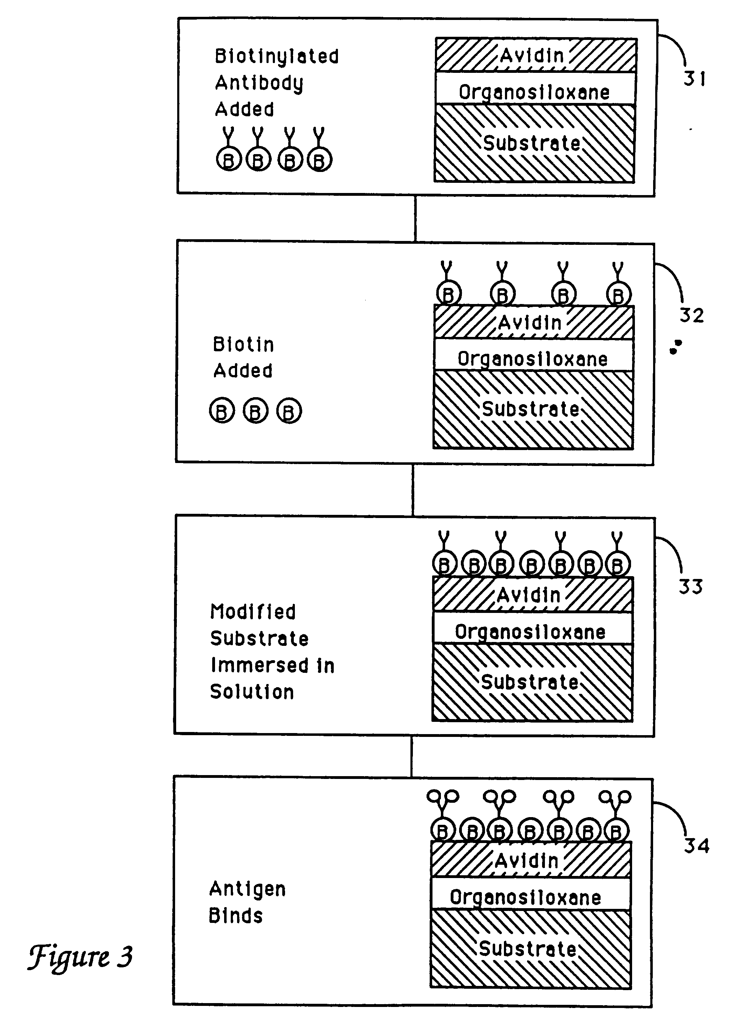

Figure 3 is a schematic representation of the process for attaching a biotinylated antibody to the avidin-coated substrate in order to bind a particular antigen. The avidin-coated substrate is incubated with the biotinylated antibody in step 31. In step 32 the substrate is then washed with a blocking agent such as biotin which binds to any unoccupied sites on the avidin layer. Other blocking agents, such as glycine, can be used. When the substrate is immersed in sample solution in step 33, antigen binds to the surface via the antibody molecule, as shown in step, 34.

The immobilization process according to the invention is further explained hereinafter by reference to the following example.

### Example 1

### Modification of a Silica Surface with Biotinylated Antibody

1. Silanization of silica substrate.
   Prepare a 10% solution of 3-glycidoxypropyltrimethoxysilane (GOPS), pH 3.0, using 2.5 ml GOPS (Aldrich Chemical Co.), 20 ml isopropanol, 2.5 ml H₂O, and 1 ml acetic acid. Expose a silica substrate pretreated with SiO₂ to the solution. Allow the solution to hydrolyze 1 hour then add 0.25 ml triethylamine (Aldrich Chemical Co.), as catalyst. Allow 1 hour for binding. Rinse 3-5X with distilled water. Allow the surface to dry *in vacuo* or under helium, or in a mechanical oven at 110°C for 10 minutes.
2. Oxidation of epoxide or diol groups on GOPS by periodate oxidation.
   Prepare a 0.1% periodate solution with 1 g.H₅IO₆, 200 ml H₂O, 800 ml acetic acid. Incubate with the silanized substrate 30 minutes at room temperature. Wash with water.
3. Incubation of substrate with avidin.
   Incubation the washed substrate with a solution of avidin in borate buffered saline (BBS), pH 8.5, at a concentration of 0.1 mg/ml. Mix by gentle inversion at 4°C for 20-24 hours.
4. Reduction of Schiff's base to a stable reduction product.
   Following the incubation, add NaCNBH₄ at three 15 minute intervals for a final concentration of 0.01M NaCNBH₄. Rinse with BBS.
5. Binding of biotinylated antibody.
   Incubate avidin-layered substrate with biotin-conjugated antibody in PBS for 30 minutes at room temperature. Wash with PBS.

A surface layered with avidin can be customized by the user for widely different applications. Basically, the procedure utilizes a mixed conjugate comprising a biologically active molecule attached chemically to a ligand whereby the resultant product retains both its biological activity and its affinity for a ligand-binding protein. Any chemical pair that exhibits strong binding affinity toward one another is a candidate. For example, biotinylated antibodies bind antigens, biotinylated polynucleotide strands bind DNA, cDNA or RNA, biotinylated DNA-binding proteins bind regulatory sequences of genes, biotinylated Protein G and Protein A bind immunoglobulins, biotinylated lectins bind sugars, biotinylated enzymes bind their substrates, cofactors or inhibitors, and biotinylated cell receptor proteins bind hormones, neurotransmitters, or viruses. Any genetically engineered or chemically modified biotin can be substituted for biotin in the conjugate or as blocking agent as long as the avidin binding site remains available.

The method of the invention can be used on diverse substrates in accordance with the particular application. When purification is the objective, chromatographic support matrices, or silica beads can be used. Glass tubes, petri dishes, or other labware treated according to the method of the invention can be used to introduce time-saving steps and convenience to standard laboratory procedures.

Alternative methods of attachment of the avidin layer can be used. For example, an organosilane coupling agent whose nonhydrolyzable organic radical contains an amine group, (γ-aminopropyl)triethoxysilane (γ-APTES), can be reacted with glutaraldehyde and then with avidin. Some substrates, such as Sepharose, can be activated and attached directly to avidin.

The device of the invention accommodates any type of gravimetric sensor. Gravimetric sensors utilizing piezoelectric crystals include Rayleigh surface acoustic wave devices and Lamb acoustic wave devices as well as the surface transverse wave device.

While avidin and biotinylated compounds are convenient binding substances to use because of their widespread availability, other reciprocal binding substances can be used according to the method of the invention A lectin used as a first binding layer and a glycosylated receptor protein as its reciprocal binding layer will be applicable to the immobilization of virus particles, growth factors, hormones and other cell modulators. Alternatively, protein A or protein G can be incorporated in the first binding layer with antibody as the second binding layer so that the concentration of an antigen can be measured.

## Claims

1. A method of rendering a substrate surface (114) of a mass sensor (100) specific for the binding of a selected substance (124), said method comprising the steps of:
attaching an organosilane coupling agent (116) to said substrate surface;
attaching a first binding layer (118) to said organosilane coupling agent (116), said first binding layer (118) having first layer high affinity binding sites available; and subsequently
attaching a second binding layer (120) to said first layer high affinity binding sites through reciprocal high affinity binding sites on said second binding layer (120), said second binding layer (120) also having second layer binding sites available for the binding of said selected substance (124).

2. The method of Claim 1 further comprising a step of, after attaching said second binding layer (120), washing with a blocking agent.

3. The method of Claim 2 wherein said blocking agent has high affinity binding sites where are reciprocal to said first layer (118) high affinity binding sites.

4. The method of Claim 1,2 or 3 wherein said first binding layer (118) is selected from the group consisting of avidin, streptavidin, genetically engineering avidin, genetically engineered streptavidin, derivatized avidin, and derivatized streptavidin; and said second binding layer (120) is a biotinylated compound.

5. The method of Claim 4 wherein said blocking agent is biotin.

6. The method of any preceding claim, wherein the step of attaching a first binding layer (118) is carried out in conjunction with manufacturing said mass sensor (100), and the step of attaching a second binding layer (120) is carried out just prior to use of said mass sensor (100).

7. A mass sensor (100) for measuring the mass of an analyte in solution, said sensor comprising:
a substrate having a surface (114) to which said analyte is to be attached;
an organosilane coupling layer (116) bound to said surface (114), said organosilane coupling layer (116) having a functional group;
a ligand-binding layer (118) attached to said surface by means of said functional group said ligand-binding layer having high affinity ligand-binding sites for binding a ligand; and
a ligand-bearing layer (120) strongly adhering through said ligand to said ligand-binding sites of said ligand-binding layer (118), said ligand-bearing layer (120) having second binding sites that are chemically selective for said analyte, whereby said analyte is attached to said surface (114) by means of said ligand-bearing layer (120), said ligand-binding layer (118) and said organosilane coupling layer (116) so that said mass can be determined by said sensor (100).

8. A mass sensor according to claim 7 which is a surface transverse wave (STW) mass sensor, further comprising:
a signal generator (121) for generating a periodic signal;
an input transducer (112) for converting said signal into an acoustic wave which is transmitted along said substrate;
an output transducer (113) for converting said acoustic wave into a received signal, said received signal being responsive to the mass of said analyte attached to said surface (114); and
signal processor means (125) for analyzing said received signal to determine said mass.

9. A mass sensor according to claim 7 or 8, wherein said ligand-binding layer (118) is selected from the group consisting of avidin, streptavidin, genetically engineered avidin, genetically engineered streptavidin, derivatized avidin, and derivatized streptavidin; and said ligand-bearing layer (120) is a biotinylated compound.

10. A mass sensor according to claim 9, wherein said biotinylated compound is a biotinylated antibody, whereby said device binds the associated antigen.

11. A mass sensor according to claim 9, wherein said biotinylated compound is a biotinylated fragment of one strand of polynucleotide, whereby said device binds fragments of polynucleotide strands that are complementary to said biotinylated fragment.

12. A mass sensor according to claim 9, wherein said biotinylated compound is selected from the group consisting of proteins that bind immunoglobulins, whereby said mass sensor binds immunoglobulins.

13. a mass sensor according to claim 12, wherein said biotinylated compound is selected from the group comprising biotinylated Protein A and biotinylated Protein G.

14. A mass sensor according to claim 7 or 8, wherein said ligand-binding layer (118) is a lectin, said lectin having binding affinity for selected sugars, and said ligand-bearing layer (120) is a glycosylated receptor protein with said selected sugars attached.

## Patentansprüche

1. Ein Verfahren, um eine Substratoberfläche (114) eines Massensensors (100) für das Binden einer ausgewählten Substanz (124) spezifisch zu machen, mit folgenden Schritten:
Anbringen eines Organosilan-Kopplungsmittels (116) an der Substratoberfläche;
Anbringen einer ersten Bindungsschicht (118) an dem Organosilan-Kopplungsmittel (116), wobei die erste Bindungsschicht (118) Erste-Schicht-Bindungsstellen mit hoher Affinität verfügbar hat; und anschließend
Anbringen einer zweiten Bindungsschicht (120) an den Erste-Schicht-Bindungsstellen mit hoher Affinität durch reziproke Bindungsstellen mit hoher Affinität auf der zweiten Bindungsschicht (120), wobei die zweite Bindungsschicht (120) ebenfalls Zweite-Schicht-Bindungsstellen für das Binden der ausgewählten Substanz (124) verfügbar hat.

2. Das Verfahren gemäß Anspruch 1, das ferner den Schritt des Waschens mit einem Blockierungsmittel aufweist, nachdem die zweite Bindungsschicht (120) angebracht worden ist.

3. Das Verfahren gemäß Anspruch 2, bei dem das Blockierungsmittel Bindungsstellen mit hoher Affinität aufweist, welche zu den Erste-Schicht- (118) Bindungsstellen mit hoher Affinität reziprok sind.

4. Das Verfahren gemäß Anspruch 1, 2 oder 3, bei dem die erste Bindungsschicht (118) aus der Gruppe ausgewählt ist, die aus Avidin, Streptavidin, genetisch entworfenem Avidin, genetisch entworfenem Streptavidin, derivatisiertem Avidin und derivatisiertem Streptavidin besteht, wobei die zweite Bindungsschicht (120) eine biotinilierte Verbindung ist.

5. Das Verfahren gemäß Anspruch 4, bei dem das Blockierungsmittel Biotin ist.

6. Das Verfahren gemäß einem beliebigen der vorhergehenden Ansprüche, bei dem der Schritt des Anbringens einer ersten Bindungsschicht (118) in Verbindung mit der Herstellung des Massensensors (100) ausgeführt wird, während der Schritt des Anbringens einer zweiten Bindungsschicht (120) direkt vor der Verwendung des Massensensors (100) ausgeführt wird.

7. Ein Massensensor (100) zum Messen der Masse eines Analyts in einer Lösung mit folgenden Merkmalen:
einem Substrat mit einer Oberfläche (114), an der der Analyt angebracht werden soll;
einer Organosilan-Kopplungsschicht (116), die an der Oberfläche (114) gebunden ist, wobei die Organosilan-Kopplungsschicht (116) eine Funktionsgruppe aufweist;
einer Ligand-Bindungsschicht (118), die mittels der Funktionsgruppe an der Oberfläche angebracht ist, wobei die Ligand-Bindungsschicht Ligand-Bindungsstellen mit hoher Affinität zum Binden eines Ligands aufweist; und
einer Ligand-Trägerschicht (120), die durch das Ligand an den Ligand-Bindungsstellen der Ligand-Bindungsschicht (118) stark haftet, wobei die Ligand-Trägerschicht (120) zweite Bindungsstellen aufweist, die für das Analyt chemisch selektiv sind, wodurch das Analyt an der Oberfläche (114) mittels der Ligand-Trägerschicht (120), der Ligand-Bindungsschicht (118) und der Organosilan-Kopplungsschicht (116) angebracht wird, derart, daß die Masse durch den Sensor (100) bestimmt werden kann.

8. Ein Massensensor gemäß Anspruch 7, welcher ein Oberflächen-Transversalwellen-Massensensor (STW-Massensensor) ist, der ferner folgende Merkmale aufweist:
einen Signalgenerator (121) zum Erzeugen eines periodischen Signals;
einen Eingangswandler (112) zum Umwandeln des Signals in eine akustische Welle, welche entlang des Substrats übertragen wird;
einen Ausgangswandler (113) zum Umwandeln der akustischen Welle in ein empfangenes Signal, wobei das empfangene Signal auf die Masse des Analyts, der an der Oberfläche (114) angebracht ist, anspricht; und
eine Signalverarbeitungseinrichtung (125) zum Analysieren des empfangenen Signals, um die Masse zu bestimmen.

9. Ein Massensensor gemäß Anspruch 7 oder 8, bei dem die Ligand-Bindungsschicht (118) aus der Gruppe ausgewählt wird, die aus Avidin, Streptavidin, genetisch entworfenem Avidin, genetisch entworfenem Streptavidin, derivatisiertem Avidin und derivatisiertem Streptavidin besteht, und wobei die Ligand-Trägerschicht (120) eine biotinilierte Verbindung ist.

10. Ein Massensensor gemäß Anspruch 9, bei dem die biotinilierte Verbindung ein biotinilierter Antikörper ist, wodurch das Gerät das zugeordnete Antigen bindet.

11. Ein Massensensor gemäß Anspruch 9, bei dem die biotinilierte Verbindung ein biotiniliertes Fragment eines Strangs eines Polynucleotids ist, wodurch das Gerät Fragmente von Polynucleotidsträngen bindet, die zum dem biotinilierten Fragment komplementär sind.

12. Ein Massensensor gemäß Anspruch 9, bei dem die biotinilierte Verbindung aus der Gruppe ausgewählt ist, die aus Proteinen, die Immonoglobuline binden, besteht, wodurch der Massensensor Immonoglobuline bindet.

13. Ein Massensensor gemäß Anspruch 12, bei dem die biotinilierte Verbindung aus der Gruppe ausgewählt ist, die biotiniliertes Protein A und biotiniliertes Protein G aufweist.

14. Ein Massensensor gemäß Anspruch 7 oder 8, bei dem die Ligand-Bindungsschicht (118) ein Lectin ist, wobei das Lectin eine Bindungsaffinität für ausgewählte Zucker aufweist, und die Ligand-Trägerschicht (120) ein glycosyliertes Rezeptorprotein ist, an dem die ausgewählten Zucker angebracht sind.

## Revendications

1. Une méthode permettant de rendre une surface de phase solide (114) d'un capteur de masse (100) spécifique pour lier une substance sélectionnée (124), ladite méthode comprenant les étapes consistant :
à attacher un agent de pontage organosilane (116) à ladite surface de la phase solide ;
à attacher une première couche liante (118) au dit agent de pontage organosilane (116), ladite première couche liante (118) disposant de sites de liaison présentant une forte affinité avec la première couche ; et ultérieurement
à attacher une seconde couche liante (120) aux dits premiers sites de liaison présentant une forte affinité avec la première couche via des sites de liaison présentant une forte affinité réciproque sur ladite seconde couche liante (120), ladite seconde couche liante (120) disposant également de sites de liaison avec ladite seconde couche pour lier ladite substance sélectionnée (124),

2. La méthode selon la Revendication 1, comprenant en outre une étape consistant à, après avoir attaché ladite seconde couche liante (120), rincer avec un agent de blocage.

3. La méthode selon la Revendication 2, dans laquelle ledit agent de blocage comporte des sites de liaison qui présentent une forte affinité et qui sont complémentaires avec lesdits sites de liaison présentant une forte affinité avec ladite première couche (118).

4. La méthode selon la Revendication 1, 2 ou 3, dans laquelle ladite première couche liante (118) est sélectionnée parmi le groupe composé d'avidine, de streptavidine, d'avidine modifiée génétiquement, de streptavidine modifiée génétiquement, d'avidine dérivée et de streptavidine dérivée ; et ladite seconde couche liante (120) est un composé biotiné.

5. La méthode selon la Revendication 4, dans laquelle ledit agent de blocage est de la biotine.

6. La méthode selon l'une quelconque des Revendications précédentes, dans laquelle l'étape consistant à attacher une première couche liante (118) est réalisée en conjonction avec la fabrication du dit capteur de masse (100), et l'étape consistant à attacher une seconde couche liante (120) est réalisée juste avant d'utiliser ledit capteur de masse (100).

7. Un capteur de masse (100) permettant de mesurer la masse d'un produit ajouté en solution, ledit capteur comprenant :
une phase solide présentant une surface (114) à laquelle ledit produit ajouté doit être attaché ;
une couche de pontage organosilane (116) liée à ladite surface (114), ladite couche de pontage organosilane (116) présentant un groupe fonctionnel ;
une couche liant un ligand (118) attachée à ladite surface au moyen du dit groupe fonctionnel, ladite couche liant un ligand présentant des sites de liaison de ligand présentant une forte affinité pour lier un ligand ; et
une couche supportant un ligand (120) qui adhère fortement via ledit ligand aux dits sites de liaison de ligand de ladite couche liant un ligand (118), ladite couche supportant un ligand (120) présentant des seconds sites de liaison qui montrent une sélectivité chimique pour ledit produit ajouté, suite à quoi ledit produit ajouté est attaché à ladite surface (114) au moyen de ladite couche supportant un ligand (120), de ladite couche liant un ligand (118) et de ladite couche de pontage organosilane (116) de sorte que ladite masse peut être déterminée par ledit capteur (100).

8. Un capteur de masse selon la Revendication 7, qui est un capteur de masse à ondes transversales de surface, comprenant en outre :
un générateur de signal (121) permettant de générer un signal périodique ;
un transducteur d'entrée (112) permettant de convertir ledit signal en une onde acoustique qui est transmise le long de ladite phase solide ;
un transducteur de sortie (113) permettant de convertir ladite onde acoustique en un signal reçu, ledit signal reçu étant fonction de la masse du dit produit ajouté attaché à ladite surface (114) ; et
un moyen de traitement du signal (125) permettant d'analyser ledit signal reçu pour déterminer ladite masse.

9. Un capteur de masse selon la Revendication 7 ou 8, dans lequel ladite couche liant un ligand (118) est sélectionnée parmi le groupe composé d'avidine, de streptavidine, d'avidine modifiée génétiquement, de streptavidine modifiée génétiquement, d'avidine dérivée et de streptavidine dérivée ; et ladite seconde couche supportant un ligand (120) est un composé biotiné.

10. Un capteur de masse selon la Revendication 9, dans lequel ledit composé biotiné est un anticorps biotiné, suite à quoi ledit dispositif lie l'antigène associé.

11. Un capteur de masse selon la Revendication 9, dans lequel ledit composé biotiné est un fragment biotiné d'un brin de polynucléotide, suite à quoi ledit dispositif lie des fragments des brins du polynucléotide qui sont complémentaires au dit fragment biotiné.

12. Un capteur de masse selon la Revendication 9, dans lequel ledit composé biotiné est sélectionné parmi le groupe composé de protéines qui lient des immunoglobulines, suite à quoi ledit capteur de masse lie des immunoglobulines.

13. Un capteur de masse selon la Revendication 12, dans lequel ledit composé biotiné est sélectionné parmi le groupe composé de Protéine A biotinée et de Protéine G biotinée.

14. Un capteur de masse selon la Revendication 7 ou 8, dans lequel ladite couche liant un ligand (118) est une lectine, ladite lectine présentant une affinité de liaison pour des sucres sélectionnés, et ladite couche supportant un ligand (120) est une protéine réceptrice glycosylée avec les dits sucres sélectionnés attachés.
